# EUROPEAN PATENT SPECIFICATION

(11) **EP 4 358 821 B1**
(45) Date of publication and mention of the grant of the patent: **15.04.2026**
(21) Application number: 22734073.4
(22) Date of filing: 22.06.2022
(51) Int. Cl.: A61B 5/00, A61B 5/103, A61B 5/107, G06V 40/16

(54) **A METHOD OF DEMONSTRATING AN EFFECT OF A COSMETIC PROCEDURE ON A SUBJECT AS WELL AS A CORRESPONDING ANALYSING APPARATUS**
VERFAHREN ZUM NACHWEIS DER WIRKUNG EINES KOSMETISCHEN VERFAHRENS AUF EINE PERSON SOWIE ENTSPRECHENDE ANALYSEVORRICHTUNG
PROCÉDÉ DE DÉMONSTRATION DE L'EFFET D'UNE PROCÉDURE ESTHÉTIQUE SUR UN SUJET, ET APPAREIL D'ANALYSE CORRESPONDANT

(30) Priority: 23.06.2021 NL 2028519
(43) Date of publication of application: 01.05.2024
(73) Proprietor: Symae Technologies Holding B.V., 5631 ND Eindhoven (NL)
(72) Inventor: ARKESTEIJN, Walter David, 5631 ND Eindhoven (NL); BUIJS, Siedse, 5631 ND Eindhoven (NL)
(74) Representative: Algemeen Octrooi- en Merkenbureau B.V.
(86) International application number: PCT/NL2022/050359
(87) International publication number: WO 2022/271024

(56) References cited:
- WO-A1-2014/139934
- WO-A1-2018/160720
- US-A1- 2018 276 883

## Description

### Technical field

The present disclosure generally relates to the field of cosmetic procedures and, more specific, to a method and an apparatus for demonstrating the effect of a cosmetic procedure.

### Background

A cosmetic surgery may be performed to enhance overall cosmetic appearance of a subject by reshaping and adjusting normal anatomy to make it visually more appealing.

Different types of cosmetic surgeries exist. One of the procedures relates to Injectables. Injectables such as Dermal Fillers or Neuromodulators may be used to reduce the visibility of skin folds, wrinkles or change the contours or surface structure of the skin. The end result of such treatment is a smoother or younger looking skin.

Another procedure relates to a so-called facelift. A facelifts may repair sagging, loose, drooping, or wrinkled skin on the face of subject. During this procedure, facial tissues may be lifted, excess skin removed, and skin replaced over repositioned contours. Neck lifts may also be performed in conjunction with facelifts. Other procedures with respect to facelifts include nose reshaping, forehead lifts, or eyelid surgery, as an example.

These kinds of cosmetic procedures may have a very noticeable effect, but may also have a subtle effect. Or at least an effect that may not be very noticeable in all kinds of different object observation and illumination scenarios.

Following the above, it is sometimes difficult to demonstrate the effect of such a cosmetic procedure. Prior art techniques of demonstrating an effect of a cosmetic procedure are known from US 2018/276883A1.

### Summary

It is an object of the present disclosure to provide for a method for efficiently demonstrating the effect of a cosmetic procedure on a subject.

In a first aspect of the present disclosure, there is provided a method of demonstrating an effect of a cosmetic procedure on a subject, by using an analysing apparatus, wherein said method comprises the steps of:
- providing lighting to said subject using a lighting setting;
- taking at least three photos of said subject after said cosmetic procedure has been performed on said subject, wherein for each of said at least three photos a different lighting setting is used;
- creating, by said analysing apparatus, an image have three dimensional based meta data, wherein said three dimensional based meta data is determined by:
   - calculating a plurality of normals of said subject using said at least three photos, thereby obtaining curvature information of said subject;
   - implementing a skin model, wherein said skin model is based on a colour and a shining value of a skin of said subject;
- demonstrating, by said analysing apparatus, the effect of said cosmetic procedure by providing said image having said three dimensional based meta data in a virtual environment, and by differentiating in light provided in said virtual environment to said image having said three dimensional based meta data.

In accordance with the present disclosure, providing lighting to the subject may include the location of the lighting relative to the subject. This would have the effect that light may be incoming from the left hand side, from the right hand side, from the top hand side, from the bottom hand side, or any other direction. This may also include the colour of the light. The inventors have found that it may be desirable to include different colours as some cosmetic procedures may be best demonstrated under a particular colour light. Another option is the type of lighting source, for example a beam directional lighting source, a so-called point lighting source, a regular lighting source like a 230V lamp or dedicated Light Emitting Diodes, LEDs, or anything alike.

The subject is typically a human being, for example a man or a woman. The present disclosure is not limited to a particular are of the subject, but, in most cases, the cosmetic procedures are performed somewhere on the head of the subject. For example the face.

The present disclosure defines that at least three photos are taken from the subject, wherein for each photo a different lighting setting is used. That is, the light may be placed or oriented differently with respect to the subject, or a different kind of colour of lighting is used or anything alike. The inventors have found that at least three different photos with different lighting settings are required for reliably determining the three dimensional meta data of a resulting image as will be described in more detail later below.

In a next step, the image having three dimensional based meta data is created. The final image may thus resemble the previously taken images. However, the meta data is new and allows the image to be viewed with different lighting settings. There is no need to construe a full 3D image of the subject, it is sufficient to construe the three dimensional based meta data, as the meta data is sufficient for accurately resembling the image in different types of lighting.

The plurality of normals of the subject may be considered as the curvature of the subject. It may resemble how the subject is deformed in space. This may help in aiding the determination of how particular light will reflect from the skin. In an example, normals are calculated for each pixel of the image such that very detailed information of the subject is made available. Normals may also be calculated for other area sized, for example 3x3 pixels, or 5x5 pixels or 10x10 pixels or anything alike.

Further, a skin model is implemented, and a colour calue and a shining value of the skin of the subject may be entered into the skin model. The skin model may also be used for determining, for example, the shining aspects of the skin, the reflection of the skin or anything alike - for different lighting scenarios.

Finally, the effect of the cosmetic procedure may be demonstrated by providing the image having three dimensional based meta data in a virtual environment. The environment may, for example, be viewed on a display screen like a monitor, television or tablet.

Demonstrating may mean highlighting the specific cosmetic procedure, or indicating a difference in the appearance of the specific cosmetic procedure in different lighting or anything alike.

In an example, the method further comprises the step of receiving, by said analysing apparatus, said colour and shining value of said skin of said subject.

The analysing apparatus may comprise a receiving unit arranged for receiving the colour and shining value of the skin of the subject. A physician may, for example, input these values into the analysing apparatus via the corresponding receiving unit.

In a further example, the subject is a face of a human being. Other options may include other parts of the human being.

In yet another example, the step of taking at least three photos comprises taking at least six photos of said subject.

In a further example, the method comprises the step of providing a three dimensional measurement of said subject using a three dimensional measurement system, and wherein said three dimensional based meta data that is created in said step of creating, is further determined based on said provided three dimensional measurement.

In a further example, the different lighting setting comprises any of:
- a location of a lighting source;
- a light direction of said lighting source;
- a number of simultaneously used lighting sources;
- a colour of said lighting source.

In a further example, the step of taking at least three photos of said subject comprises any of:
- taking said at least three photos of said subject homogenously, such that an orientation of said subject remains constant over said at least three photos;
- taking said at least three photos of said subject heterogeneously, such that an orientation of said subject varies over said at least three photos.

In a preferred example, the subject may not move too much when taking the different photo's. This improves the process of calculating the normals. The subject should, preferably, stay as static as possible during the photo taking process.

In a further example, method further comprises the step of:
- receiving, by said analysing apparatus, translucency information of said skin of said subject, and wherein said three dimensional based meta data that is created in said step of creating, is further determined based on said received translucency information.

The inventors have found that translucency information may be taking into account as well when determining the properties that light has on the skin of a subject, for example reflection, shining and absorption properties.

In a second aspect of the present disclosure, there is provided an analysing apparatus for demonstrating an effect of a cosmetic procedure on a subject, wherein said analysing apparatus comprises:
- providing equipment arranged for providing lighting to said subject using a lighting setting;
- a camera unit arranged for taking at least three photos of said subject after said cosmetic procedure has been performed on said subject, wherein for each of said at least three photos a different lighting setting is used;
- a processing unit arranged for creating an image have three dimensional based meta data, wherein said three dimensional based meta data is determined by:
   - calculating a plurality of normal of said subject using said at least three photos, thereby obtaining curvature information of said subject;
   - implementing a skin model, wherein said skin model is based on a colour and a shining value of a skin of said subject;
- a demonstrating unit arranged for demonstrating the effect of said cosmetic procedure by providing said image having said three dimensional based meta data in a virtual environment, and by differentiating in light provided in said virtual environment to said image having said three dimensional based meta data.

It is noted that the advantages as explained with respect to the A method of demonstrating an effect of a cosmetic procedure are also applicable to the analysing apparatus in accordance with the present disclosure.

In an example, the apparatus further comprises:
- a receiving equipment arranged for receiving said colour and shining value of said skin of said subject.

In a further example, the subject is a face of a human being.

In another example, the camera unit is further arranged for taking at least three photos comprises taking at least six photos of said subject.

In an example, the apparatus further comprises:
- provide equipment arranged for providing a three dimensional measurement of said subject using a three dimensional measurement system, and wherein said three dimensional based meta data that is created by said processing unit, is further determined based on said provided three dimensional measurement.

In a further example, wherein the different lighting setting comprises any of:
- a location of a lighting source;
- a light direction of said lighting source;
- a number of simultaneously used lighting sources;
- a colour of said lighting source.

In yet another example, the camera unit is further arranged for any of:
- taking said at least three photos of said subject homogenously, such that an orientation of said subject remains constant over said at least three photos;
- taking said at least three photos of said subject heterogeneously, such that an orientation of said subject varies over said at least three photos.

In an even further example, the analysing apparatus comprises receiving equipment arranged for receiving translucency information of said skin of said subject, and wherein said three dimensional based meta data that is created by said processing unit, is further determined based on said received translucency information.

These and other aspects of the invention will be apparent from and elucidated with reference to the embodiment(s) described hereinafter. The invention is defined by the appended claims.

### Brief description of the Drawings

Figs. 1a-d show views of a face imaging device;
Figs. 2a-f show views of the face imaging device shown in figure 1, a display and a person's head in various positions;
Fig. 3 shows a view of the face imaging device shown in figure 1 in combination with a person's head;
Figs. 4a,b show top views of a the face imaging device;
Fig. 5 discloses a method in accordance with the present disclosure.

### Detailed description

Like parts are indicated by the same reference signs in the various figures.

Each feature disclosed with reference to the figure can also be combined with another feature disclosed in this disclosure including the claims, unless it is evident for a person skilled in the art that these features are incompatible.

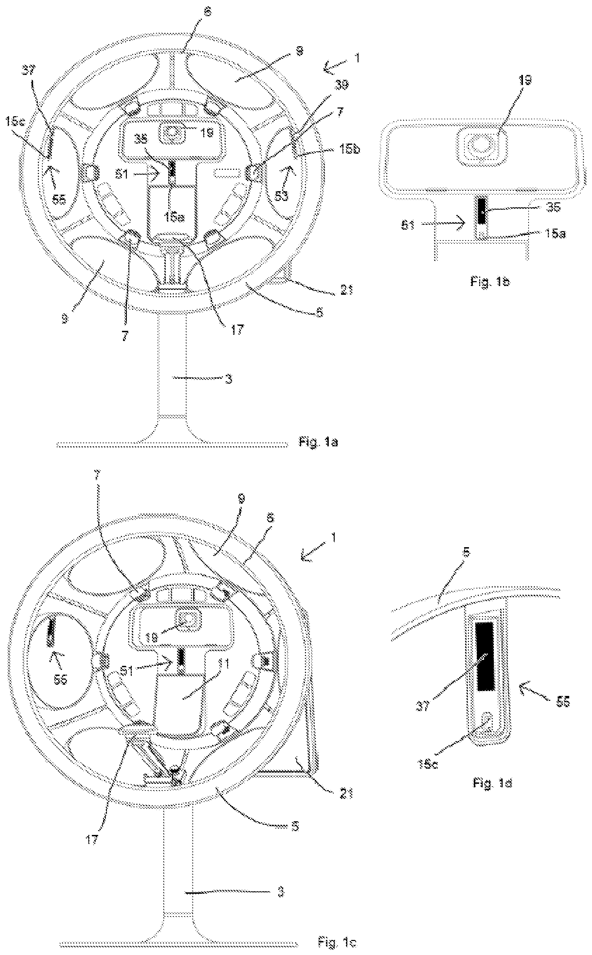

Figures 1a and 1c show a face imaging device 1, wherein figures 1b and 1d show a detail of the device. It is noted that, in accordance with the present disclosure, there is provided an analysing apparatus for demonstrating the effect of a cosmetic procedure on a subject. In an example, the subject is to be the face of a person. The text below focusses on this particular aspect. The present disclosure is, however, not limited to cosmetic procedures on a face of a subject, the cosmetic procedures may be performed on any body part of a human being.

The device 1 is a portable device comprising a base mount 3 to be placed on a support (not shown), for example a table. The device 1 comprises a casing 5 having an endless edge 6 defining an opening for a face to be imaged. In the interior of the casing 5 various light sources 7, like Light Emitting Diode LED based light sources, reflectors 9, and a mirror 11 may be arranged.

The light sources 7 may be used, for example in combination with the reflectors 9 and the mirror 11, to provide lighting to the face using a particular lighting setting. The lighting setting is aimed at, for example, the colour of light and, more particularly, the orientation of the light. That is, the direction of the light towards the subject. The orientation and/or the direction of the light may be controlled by directly amending the light sources 7, but may also be controlled by amending the mirror 11 or reflectors 9, for example.

The device 1 may be provided with a light shield (not shown) connected or to be connected to the casing 5 to be positioned in use around a person's head for blocking environmental light. As shown in more detail in figures 1b and 1d the casing 5 may comprise three sensors 15a-c for determining the actual face orientation of the face to be positioned at least partially in the opening or in front of the opening of the casing 5 on a chin support 17 of the device 1. The three sensors 15a-c may comprise a central sensor 15a and two outer sensors 15b,c.

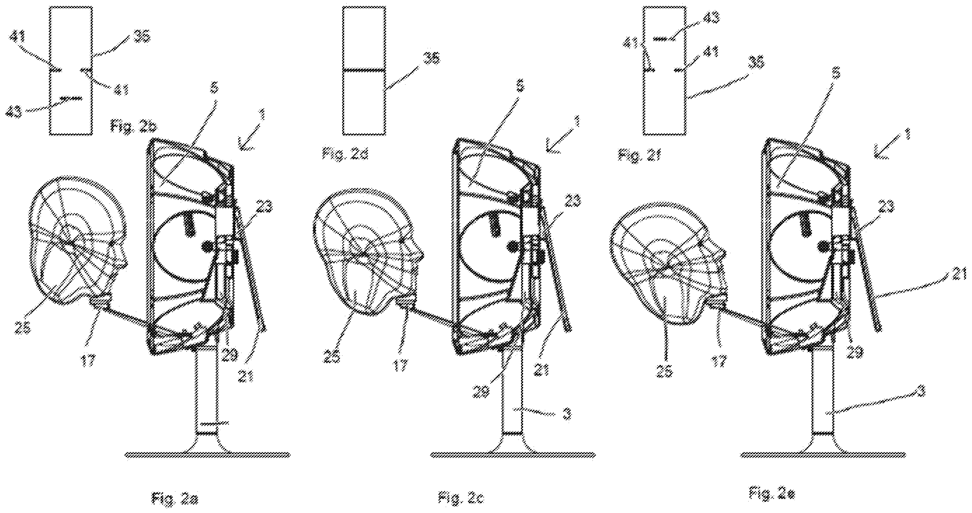

The device 1 further comprises a viewing opening/hole 19 positioned near the central sensor 15a for a camera. The camera may be a camera of a tablet computer 21. The face imaging device 1 may also comprise a fixed camera (not shown). The device 1 may further comprise a camera holding unit 23 (figure 2a) for holding the camera of the tablet computer 21 in a releasable manner in a predetermined position in the device 1 to provide at least one image of the face. The light sources 7 of the face imaging device 1 may be configured to provide two or more face observation modes, for example a first mode using UltraViolet, UV, radiation to make diagnostic observations and a second mode to make a daylight observation. These observations of the face can be imaged by the camera of the tablet computer 21.

The camera is thus arranged to take at least three photos of the subject, for example after the cosmetic procedure has been performed on the subject, wherein for each of the at least three photos a different lighting setting is used. A different lighting setting is used for being able to efficiently provide the three dimensional meta data.

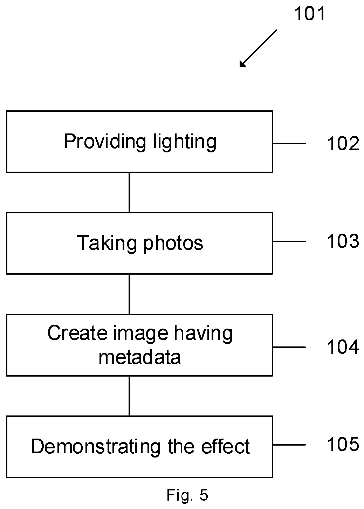

The face imaging device 1 for taking images of a face of a person may also be configured for obtaining an image of the face of the person with standardized face positioning and/or for obtaining at least a first image of the face and at least a second image of the same face for comparison, for example to compare the effects of a skin treatment performed on the face between the at least one first and the at least one second image, preferably the first and second image are taken under substantially identical conditions, for example substantially identical light conditions. At least one of the sensors 15a-c determines a face orientation of the face as shown in figures 2a-f, i.e. at least one of the sensors 15a-c determines at least one degree of freedom of a person's head 25. In addition, the chin support 17 for supporting a chin of a person's head 25 restricts head movement in three (of the total of six, see figure 5) degrees of freedom. The sensors 15a-c are configured to use a portion of the person's head or face, for example a portion of the fore head to determine at least one of the three degrees of freedom not restricted by the chin support 17. The sensors 15a-c may also determine more than one degree of freedom, for example two or three degrees of freedom as will be discussed below with reference to figure 6.

The face imaging device 1 may further comprises a processing unit arranged for creating an image having three dimensional based meta data, wherein the three dimensional based meta data is determined by calculating a plurality of normals of the subject, i.e. face, using at least three photos, thereby obtaining curvature information of the subject and by implementing a skin model, wherein the skin model is based on a colour and a shining value of the skin of the subject.

The inventors have found that the normal information of the skin as well as the so-defined shining value of the skin may aid in the process of determining the effect of the cosmetic procedure. These aspects allow an image to be created with three dimensional based meta data, wherein the meta data may be used to accurately display the image in different lighting settings.

Following the above, it is thus not necessary, in accordance with the prior art, to take photos of the subject with all possible lighting settings. The photos are used to curvature information with respect to the image, and the curvature information is used when the lighting setting is amended in the virtual environment. So, even though particular lighting settings have not been used when taking the photos, initially, the obtained meta data may be used for accurately displaying the subject in the lighting setting so desired.

As shown in figures 1a-d, 2a-f, the face imaging device 1 comprises three displays 35, 37, 39 arranged in the interior of the casing to show the person a desired face orientation, indicated by the symbols/lines 41 on the display 35 (figures 2b, 2d, 2f), and the actual face orientation of the face determined by the sensor 15a and indicated on the display 35 by the symbol/line 43 such that the person is able to position his/her face in the desired face orientation. In the device one display 35, 37, 39 and one sensor 15a-c are arranged in one unit 51, 53, 55 (figures 1a-d), i.e. a central unit 51 and two outer units 53, 55. In figure 2a the person's head is in the incorrect position as shown in the display 35 presented in figure 2b. By moving his/her head upwards, the symbol/line 43 moves upwards for example to a position as shown in figure 2f. Then, by moving his/her head downwards the symbol/line 43can be aligned with the symbols/lines 41 as shown in figure 2d which corresponds with the position of the person's head as shown in figure 2c, in which position the camera of the tablet computer 21 may take the image of the face of the person positioned in or near the opening of the casing to obtain an image of the face with standardized face positioning. The displays 35, 37, 39 may have an identical user interface. The user interface as shown in the figures 2a-2f and 6 facilitates the correct positioning of the face for taking the image of the face with standardized face positioning.

The display 35, 37, 39 is configured to show at least two associated symbols/lines 41, 43, representing at least one degree of freedom of the head of the person, wherein one 41 of the two associated symbols corresponds to the at least one degree of freedom of the desired face orientation and the other 43 of the two associated symbols corresponds to the at least one degree of freedom of the actual face orientation determined by at least one of the sensor 15a-c. By moving his/her head between positions as shown in figures 2a,c,e in the at least one degree of freedom indicated by the two associated symbols 41, 43, a user intuitively is able to position his head/face in the desired face orientation in a relatively accurate manner, i.e. as soon as the associated symbols/lines 41, 43 coincide or align an image can be taken, wherein in the position of the person's head shown in figures 2c and 2d an image may be taken to obtain the image of the face with standardized face positioning. In other words, the displays 35, 37, 39 inside the casing 5 of the device 1 are arranged to show the person a desired face orientation and the actual face orientation of the face determined by the at least one sensor 15a-c such that the person is able to position his/her face in the desired face orientation for taking at least one image of the face.

The display 35, 37, 39 may thus be configured to provide the image having the three dimensional based meta data in a virtual environment, by differentiating in light provided in the virtual environment to the image having the three dimensional based meta data.

The device 1 further comprises a processor 29 (figures 2a, 2c, 2e) configured to use the face orientation of the face determined by at least one of the sensors 15a-c to permit the device to obtain the image of the face with standardized face positioning if the face orientation of the face determined by the at least one sensor 15a-c corresponds to the desired face orientation of the face.

The processor 29 may be configured to communicate with the camera of the tablet computer 21 to instruct the camera to automatically take the image if the face orientation of the face determined by the at least one sensor corresponds to the desired face orientation of the face. Further, by automatically obtaining the second image, the face imaging device 1 itself provides the image of the face without the risk that an operator or the person himself makes an image which has a different face orientation than the desired face orientation.

The processer 29 may thus be arranged, in cooperation with any other component in the device 1, to determined or create the image having the three dimensional based meta data.

In the embodiment of the device and the display 35 as shown in figures 2a-f, the face orientation in each image of at least two images of the face to be taken by the device for comparison purposes is identical in at least four degrees of freedom to compare the first and second images for example for showing progress of a face skin treatment and/or for diagnosing skin and/or scalp conditions. Using this corresponding face orientation of the face in the first and the second image guarantees an improved comparison quality, wherein external effects can be reduced significantly. Further, identical lighting conditions between the at least two images to be compared may be preferred by using the light sources 7 in the same face observation mode and/or by using a shield shielding off in combination with the casing 5 environmental light or other external influences.

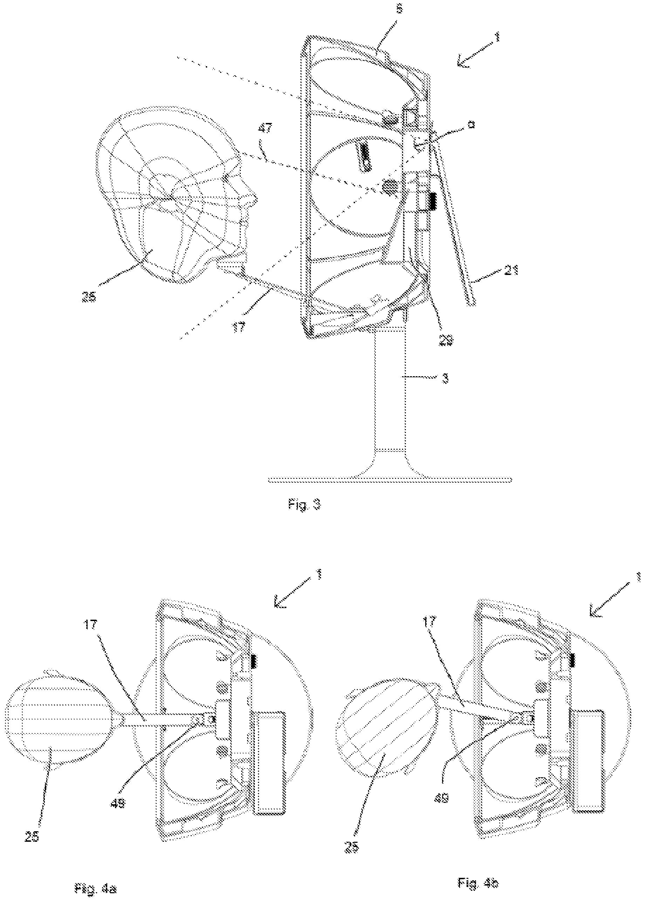

Figure 3 shows the vertical viewing angle α of the camera of the tablet computer 21 and the viewing direction 47 of the sensor 15a to determine the face orientation of the face of the person's head 25. As shown in figures 4a and 4b the chin support is pivotably connected about a pivot 49 to the device 1 to permit the chin support to move to a different position for taking images of the side face of the face of the person's head 25.

Fig. 5 discloses a method 101 in accordance with the present disclosure.

The method 101 is directed to demonstrating an effect of a cosmetic procedure on a subject, by using an analysing apparatus, wherein said method comprises the steps of:
- providing 102 lighting to said subject using a lighting setting;
- taking 103 at least three photos of said subject after said cosmetic procedure has been performed on said subject, wherein for each of said at least three photos a different lighting setting is used;
- creating 104, by said analysing apparatus, an image have three dimensional based meta data, wherein said three dimensional based meta data is determined by:
   - calculating a plurality of normals of said subject using said at least three photos, thereby obtaining curvature information of said subject;
   - implementing a skin model, wherein said skin model is based on a colour and a shining value of a skin of said subject;
- demonstrating 105, by said analysing apparatus, the effect of said cosmetic procedure by providing said image having said three dimensional based meta data in a virtual environment, and by differentiating in light provided in said virtual environment to said image having said three dimensional based meta data.

Other variations to the disclosed embodiments can be understood and effected by those skilled in the art in practicing the claimed invention, from a study of the drawings, the disclosure, and the appended claims. In the claims, the word "Comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope thereof.

## Claims

1. A computer-implemented method of demonstrating an effect of a cosmetic procedure on a subject, by using an analysing apparatus, wherein said method comprises the steps of:
- providing lighting to said subject using a lighting setting;
- taking at least three photos of said subject after said cosmetic procedure has been performed on said subject, wherein for each of said at least three photos a different lighting setting is used;
- creating, by said analysing apparatus, an image have three dimensional based meta data, wherein said three dimensional based meta data is determined by:
- calculating a plurality of normals of said subject using said at least three photos, thereby obtaining curvature information of said subject;
- implementing a skin model, wherein said skin model is based on a colour and a shining value of a skin of said subject;
- demonstrating, by said analysing apparatus, the effect of said cosmetic procedure by providing said image having said three dimensional based meta data in a virtual environment, and by differentiating in light provided in said virtual environment to said image having said three dimensional based meta data.

2. A method in accordance with claim 1, wherein said method further comprises the step of:
- receiving, by said analysing apparatus, said colour and shining value of said skin of said subject.

3. A method in accordance with any of the previous claims, wherein said subject is a face of a human being.

4. A method in accordance with any of the previous claims, wherein said step of taking at least three photos comprises taking at least six photos of said subject.

5. A method in accordance with any of the previous claims, wherein said method further comprises the step of:
- providing a three dimensional measurement of said subject using a three dimensional measurement system, and wherein said three dimensional based meta data that is created in said step of creating, is further determined based on said provided three dimensional measurement.

6. A method in accordance with any of the previous claims, wherein said a different lighting setting comprises any of:
- a location of a lighting source;
- a light direction of said lighting source;
- a number of simultaneously used lighting sources;
- a colour of said lighting source.

7. A method in accordance with any of the previous claims, wherein said step of taking at least three photos of said subject comprises any of:
- taking said at least three photos of said subject homogenously, such that an orientation of said subject remains constant over said at least three photos;
- taking said at least three photos of said subject heterogeneously, such that an orientation of said subject varies over said at least three photos.

8. A method in accordance with any of the previous claims, wherein said method further comprises the step of:
- receiving, by said analysing apparatus, translucency information of said skin of said subject, and wherein said three dimensional based meta data that is created in said step of creating, is further determined based on said received translucency information.

9. An analysing apparatus for demonstrating an effect of a cosmetic procedure on a subject, wherein said analysing apparatus comprises:
- providing equipment arranged for providing lighting to said subject using a lighting setting, wherein the lighting setting preferably comprises any of:
- a location of a lighting source;
- a light direction of said lighting source;
- a number of simultaneously used lighting sources;
- a colour of said lighting source. - a camera unit arranged for taking at least three photos of said subject after said cosmetic procedure has been performed on said subject, wherein for each of said at least three photos a different lighting setting is used;
- a processing unit arranged for creating an image have three dimensional based meta data, wherein said three dimensional based meta data is determined by:
- calculating a plurality of normals of said subject using said at least three photos, thereby obtaining curvature information of said subject;
- implementing a skin model, wherein said skin model is based on a colour and a shining value of a skin of said subject;
- a demonstrating unit arranged for demonstrating the effect of said cosmetic procedure by providing said image having said three dimensional based meta data in a virtual environment, and by differentiating in light provided in said virtual environment to said image having said three dimensional based meta data.

10. An analysing apparatus in accordance with claim 9, wherein said apparatus further comprises:
- a receiving equipment arranged for receiving said colour and shining value of said skin of said subject.

11. An analysing apparatus in accordance with any of the claims 9 - 10, wherein said subject is a face of a human being.

12. An analysing apparatus in accordance with any of the claims 9 - 11, wherein said camera unit is further arranged for taking at least three photos comprises taking at least six photos of said subject.

13. An analysing apparatus in accordance with claim 12, wherein said apparatus further comprises:
- provide equipment arranged for providing a three dimensional measurement of said subject using a three dimensional measurement system, and wherein said three dimensional based meta data that is created by said processing unit, is further determined based on said provided three dimensional measurement.

14. An analysing apparatus in accordance with any of the claims 9 - 13, wherein said camera unit is further arranged for any of:
- taking said at least three photos of said subject homogenously, such that an orientation of said subject remains constant over said at least three photos;
- taking said at least three photos of said subject heterogeneously, such that an orientation of said subject varies over said at least three photos.

15. An analysing apparatus in accordance with any of the claims 9 - 14, wherein said analysing apparatus comprises receiving equipment arranged for receiving translucency information of said skin of said subject, and wherein said three dimensional based meta data that is created by said processing unit, is further determined based on said received translucency information.

## Patentansprüche

1. Computerimplementiertes Verfahren zum Nachweis der Wirkung eines kosmetischen Verfahrens auf eine Person unter Verwendung einer Analysevorrichtung, wobei das Verfahren die folgenden Schritte umfasst:
- Bereitstellen einer Beleuchtung für die Person unter Verwendung einer Beleuchtungseinstellung;
- Aufnehmen von mindestens drei Fotos der Person, nachdem das kosmetische Verfahren an der Person durchgeführt wurde, wobei für jedes der mindestens drei Fotos eine unterschiedliche Beleuchtungseinstellung verwendet wird;
- Erzeugen, durch die Analysevorrichtung, eines Bildes mit auf drei Dimensionen basierenden Metadaten, wobei die auf drei Dimensionen basierenden Metadaten bestimmt werden durch:
- Berechnen einer Vielzahl von Normalen der Person unter Verwendung der mindestens drei Fotos, wodurch Konturinformationen der Person erhalten werden;
- Implementieren eines Hautmodells, wobei das Hautmodell auf einem Farbwert und einem Glanzwert der Haut der Person basiert;
- Nachweisen, durch die Analysevorrichtung, der Wirkung des kosmetischen Verfahrens durch Bereitstellen des Bildes mit den auf drei Dimensionen basierenden Metadaten in einer virtuellen Umgebung sowie durch Variieren der in der virtuellen Umgebung auf das Bild mit den auf drei Dimensionen basierenden Metadaten einwirkenden Beleuchtung.

2. Verfahren nach Anspruch 1, wobei das Verfahren ferner den folgenden Schritt umfasst:
- Empfangen, durch die Analysevorrichtung, des Farbwerts und des Glanzwerts der Haut der Person.

3. Verfahren nach einem der vorhergehenden Ansprüche, wobei die Person sich auf ein Gesicht eines Menschen bezieht.

4. Verfahren nach einem der vorhergehenden Ansprüche, wobei der Schritt des Aufnehmens von mindestens drei Fotos das Aufnehmen von mindestens sechs Fotos der Person umfasst.

5. Verfahren nach einem der vorhergehenden Ansprüche, wobei das Verfahren ferner den folgenden Schritt umfasst:
- Bereitstellen einer dreidimensionalen Vermessung der Person unter Verwendung eines dreidimensionalen Vermessungssystems, und wobei die in dem Schritt des Erzeugens erzeugten auf drei Dimensionen basierenden Metadaten ferner auf der bereitgestellten dreidimensionalen Vermessung basieren.

6. Verfahren nach einem der vorhergehenden Ansprüche, wobei die unterschiedliche Beleuchtungseinstellung eines der folgenden Merkmale umfasst:
- eine Position einer Lichtquelle;
- eine Lichtrichtung der Lichtquelle;
- eine Anzahl gleichzeitig verwendeter Lichtquellen;
- eine Farbe der Lichtquelle.

7. Verfahren nach einem der vorhergehenden Ansprüche, wobei der Schritt des Aufnehmens von mindestens drei Fotos der Person Folgendes umfasst:
- homogenes Aufnehmen der mindestens drei Fotos der Person, sodass eine Ausrichtung der Person über die mindestens drei Fotos hinweg konstant bleibt;
- heterogenes Aufnehmen der mindestens drei Fotos der Person, sodass eine Ausrichtung der Person über die mindestens drei Fotos hinweg variiert.

8. Verfahren nach einem der vorhergehenden Ansprüche, wobei das Verfahren ferner den folgenden Schritt umfasst:
- Empfangen, durch die Analysevorrichtung, von Transluzenzinformationen der Haut der Person, wobei die in dem Schritt des Erzeugens erzeugten auf drei Dimensionen basierenden Metadaten ferner auf den empfangenen Transluzenzinformationen basieren.

9. Analysevorrichtung zum Nachweis der Wirkung eines kosmetischen Verfahrens auf eine Person, wobei die Analysevorrichtung umfasst:
- eine Bereitstellungseinrichtung, die zum Bereitstellen einer Beleuchtung für die Person unter Verwendung einer Beleuchtungseinstellung eingerichtet ist, wobei die Beleuchtungseinstellung vorzugsweise eine der Folgenden umfasst:
- eine Position einer Lichtquelle;
- eine Lichtrichtung der Lichtquelle;
- eine Anzahl gleichzeitig verwendeter Lichtquellen;
- eine Farbe der Lichtquelle. - eine Kameraeinheit, die zum Aufnehmen von mindestens drei Fotos der Person, nachdem das kosmetische Verfahren an der Person durchgeführt wurde, eingerichtet ist, wobei für jedes der mindestens drei Fotos eine unterschiedliche Beleuchtungseinstellung verwendet wird;
- eine Verarbeitungseinheit, die zum Erzeugen eines Bildes mit auf drei Dimensionen basierenden Metadaten eingerichtet ist, wobei die auf drei Dimensionen basierenden Metadaten bestimmt werden durch:
- Berechnen einer Vielzahl von Normalen der Person unter Verwendung der mindestens drei Fotos, wodurch Konturinformationen der Person erhalten werden;
- Implementieren eines Hautmodells, wobei das Hautmodell auf einem Farbwert und einem Glanzwert der Haut der Person basiert;
- eine Demonstrationseinheit, die eingerichtet ist zum Nachweisen der Wirkung des kosmetischen Verfahrens durch Bereitstellen des Bildes mit den auf drei Dimensionen basierenden Metadaten in einer virtuellen Umgebung sowie durch Variieren der in der virtuellen Umgebung auf das Bild mit den auf drei Dimensionen basierenden Metadaten einwirkenden Beleuchtung.

10. Analysevorrichtung nach Anspruch 9, wobei die Vorrichtung ferner umfasst:
- eine Empfangseinrichtung, die zum Empfangen des Farbwerts und des Glanzwerts der Haut der Person eingerichtet ist.

11. Analysevorrichtung nach einem der Ansprüche 9 bis 10, wobei die Person sich auf ein Gesicht eines Menschen bezieht.

12. Analysevorrichtung nach einem der Ansprüche 9 bis 11, wobei die Kameraeinheit ferner zum Aufnehmen von mindestens sechs Fotos der Person eingerichtet ist.

13. Analysevorrichtung nach Anspruch 12, wobei die Vorrichtung ferner umfasst:
- eine Bereitstellungseinrichtung, die zum Bereitstellen einer dreidimensionalen Vermessung der Person unter Verwendung eines dreidimensionalen Vermessungssystems eingerichtet ist, und wobei die von der Verarbeitungseinheit erzeugten auf drei Dimensionen basierenden Metadaten ferner auf der bereitgestellten dreidimensionalen Vermessung basieren.

14. Analysevorrichtung nach einem der Ansprüche 9 bis 13, wobei die Kameraeinheit ferner für eines der Folgenden eingerichtet ist:
- homogenes Aufnehmen der mindestens drei Fotos der Person, sodass eine Ausrichtung der Person über die mindestens drei Fotos hinweg konstant bleibt;
- heterogenes Aufnehmen der mindestens drei Fotos der Person, sodass eine Ausrichtung der Person über die mindestens drei Fotos hinweg variiert.

15. Analysevorrichtung nach einem der Ansprüche 9 bis 14, wobei die Analysevorrichtung eine Empfangseinrichtung umfasst, die zum Empfangen von Transluzenzinformationen der Haut der Person eingerichtet ist, und wobei die von der Verarbeitungseinheit erzeugten auf drei Dimensionen basierenden Metadaten ferner auf den empfangenen Transluzenzinformationen basieren.

## Revendications

1. Procédé mis en œuvre par ordinateur pour démontrer l'effet d'une intervention esthétique sur un sujet, en utilisant un appareil d'analyse, dans lequel ledit procédé comprend les étapes de :
- fourniture d'un éclairage audit sujet en utilisant un réglage d'éclairage ;
- prise d'au moins trois photos dudit sujet après que ladite intervention esthétique a été effectuée sur ledit sujet, dans lequel pour chacune desdites au moins trois photos, un réglage d'éclairage différent est utilisé ;
- création, par ledit appareil d'analyse, d'une image ayant des métadonnées tridimensionnelles, dans lequel lesdites métadonnées tridimensionnelles sont déterminées par :
- le calcul d'une pluralité de normales dudit sujet en utilisant lesdites au moins trois photos, obtenant ainsi des informations de courbure dudit sujet ;
- la mise en œuvre d'un modèle de peau, dans lequel ledit modèle de peau est basé sur une couleur et une valeur de brillance de la peau dudit sujet ;
- démonstration, par ledit appareil d'analyse, de l'effet de ladite intervention cosmétique en fournissant ladite image ayant lesdites métadonnées tridimensionnelles dans un environnement virtuel, et en différenciant la lumière fournie dans ledit environnement virtuel à ladite image ayant lesdites métadonnées tridimensionnelles.

2. Procédé selon la revendication 1, dans lequel ledit procédé comprend en outre l'étape de :
- réception, par ledit appareil d'analyse, de ladite couleur et de ladite valeur de brillance de ladite peau dudit sujet.

3. Procédé selon l'une des revendications précédentes, dans lequel ledit sujet est le visage d'un être humain.

4. Procédé selon l'une des revendications précédentes, dans lequel ladite étape de prise d'au moins trois photos comprend la prise d'au moins six photos dudit sujet.

5. Procédé selon l'une des revendications précédentes, dans lequel ledit procédé comprend en outre l'étape de :
- fourniture d'une mesure tridimensionnelle dudit sujet en utilisant un système de mesure tridimensionnelle, et dans lequel lesdites métadonnées tridimensionnelles qui sont créées dans ladite étape de création sont en outre déterminées sur la base de ladite mesure tridimensionnelle fournie.

6. Procédé selon l'une des revendications précédentes, dans lequel ledit réglage d'éclairage différent comprend l'un quelconque parmi :
- un emplacement d'une source d'éclairage ;
- une direction de lumière de ladite source d'éclairage ;
- un nombre de sources d'éclairage utilisées simultanément ;
- une couleur de ladite source d'éclairage.

7. Procédé selon l'une des revendications précédentes, dans lequel ladite étape de prise d'au moins trois photos dudit sujet comprend l'une quelconque parmi :
- la prise desdites au moins trois photos dudit sujet de manière homogène, de sorte que l'orientation dudit sujet reste constante sur lesdites au moins trois photos ;
- la prise desdites au moins trois photos dudit sujet de manière hétérogène, de sorte que l'orientation dudit sujet varie sur lesdites au moins trois photos.

8. Procédé selon l'une des revendications précédentes, dans lequel ledit procédé comprend en outre l'étape de :
- réception, par ledit appareil d'analyse, d'informations de translucidité de ladite peau dudit sujet, et dans lequel lesdites métadonnées tridimensionnelles qui sont créées dans ladite étape de création sont en outre déterminées sur la base desdites informations de translucidité reçues.

9. Appareil d'analyse pour démontrer l'effet d'une intervention cosmétique sur un sujet, dans lequel ledit appareil d'analyse comprend :
- un équipement de fourniture agencé pour fournir un éclairage audit sujet en utilisant un réglage d'éclairage, dans lequel le réglage d'éclairage comprend de préférence l'un quelconque parmi :
- un emplacement d'une source d'éclairage ;
- une direction de lumière de ladite source d'éclairage ;
- un nombre de sources d'éclairage utilisées simultanément ;
- une couleur de ladite source d'éclairage.
- une unité de caméra agencée pour prendre au moins trois photos dudit sujet après que ladite intervention cosmétique a été effectuée sur ledit sujet, dans lequel, pour chacune desdites au moins trois photos, un réglage d'éclairage différent est utilisé ;
- une unité de traitement agencée pour créer une image ayant des métadonnées tridimensionnelles, dans lequel lesdites métadonnées tridimensionnelles sont déterminées par :
- le calcul d'une pluralité de normales dudit sujet en utilisant lesdites au moins trois photos, obtenant ainsi des informations de courbure dudit sujet ;
- la mise en œuvre d'un modèle de peau, dans lequel ledit modèle de peau est basé sur une couleur et une valeur de brillance de la peau dudit sujet ;
- une unité de démonstration agencée pour démontrer l'effet de ladite intervention cosmétique en fournissant ladite image ayant lesdites métadonnées tridimensionnelles dans un environnement virtuel, et en différenciant la lumière fournie dans ledit environnement virtuel à ladite image ayant lesdites métadonnées tridimensionnelles.

10. Appareil d'analyse selon la revendication 9, dans lequel ledit appareil comprend en outre :
- un équipement de réception agencé pour recevoir ladite couleur et ladite valeur de brillance de ladite peau dudit sujet.

11. Appareil d'analyse selon l'une des revendications 9 et 10, dans lequel ledit sujet est le visage d'un être humain.

12. Appareil d'analyse selon l'une des revendications 9 à 11, dans lequel ladite unité de caméra est en outre agencée pour prendre au moins trois photos comprend la prise d'au moins six photos dudit sujet.

13. Appareil d'analyse selon la revendication 12, dans lequel ledit appareil comprend en outre :
- un équipement de fourniture agencé pour fournir une mesure tridimensionnelle dudit sujet en utilisant un système de mesure tridimensionnelle, et dans lequel lesdites métadonnées tridimensionnelles qui sont créées par ladite unité de traitement sont en outre déterminées sur la base de ladite mesure tridimensionnelle fournie.

14. Appareil d'analyse selon l'une des revendications 9 à 13, dans lequel ladite unité de caméra est en outre agencée pour l'une quelconque de ce qui suit :
- la prise desdites au moins trois photos dudit sujet de manière homogène, de sorte que l'orientation dudit sujet reste constante sur lesdites au moins trois photos ;
- la prise desdites au moins trois photos dudit sujet de manière hétérogène, de sorte que l'orientation dudit sujet varie sur lesdites au moins trois photos.

15. Appareil d'analyse selon l'une des revendications 9 à 14, dans lequel ledit appareil d'analyse comprend un équipement de réception agencé pour recevoir des informations de translucidité de ladite peau dudit sujet, et dans lequel lesdites métadonnées tridimensionnelles qui sont créées par ladite unité de traitement sont en outre déterminées sur la base desdites informations de translucidité reçues.
